# EUROPEAN PATENT APPLICATION

(11) **EP 1 445 324 A1**
(43) Date of publication of application: **11.08.2004**
(21) Application number: 02781744.4
(22) Date of filing: 07.11.2002
(51) Int. Cl.: C12P 17/06, C12P 7/22, C07D 311/20, C07D 311/58, C07C 309/66, C07C 39/24

(54) **PROCESS FOR PRODUCING OPTICALLY ACTIVE CHROMAN DERIVATIVE AND INTERMEDIATE**

(30) Priority: 09.11.2001 JP 2001345232
(71) Applicant: KANEKA CORPORATION, Osaka-shi, Osaka 530-8288 (JP)
(72) Inventor: MITSUDA, M. c/o KANEKA CORPORATION, Takasago Plant, Takasago-shi, Hyogo 676-8688 (JP); TANAKA, T., c/o KANEKA CORPORATION, Takasago Plant, Takasago-shi, Hyogo 676-8688 (JP); YASOHARA, Yoshihiko, c/o KANEKA CORPORATION, Takasago-shi, Hyogo 676-8688 (JP)
(74) Representative: VOSSIUS & PARTNER
(86) International application number: PCT/JP2002/011592
(87) International publication number: WO 2003/040382

(57) **Abstract**

A process for easily producing various optically active chroman derivatives that are useful as pharmaceutical intermediates from inexpensive starting materials is provided.

Cyclic hemiacetal (1) obtained from dihydrocoumarin through one step is asymmetrically reduced to produce an optically active halohydrin derivative (3), and the optically active halohydrin derivative (3) is cyclized to produce an optically active chroman derivative (13):

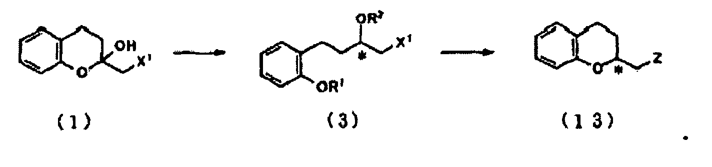

## Description

### Technical Field

The present invention relates to a process for producing optically active chroman derivatives useful as intermediates for serotonin receptor agonists, e.g., intermediates disclosed in European Patent No. 707007, and to intermediates therefor.

### Background Art

The following techniques have been known as processes for producing optically active chroman derivatives represented by general formula (13): 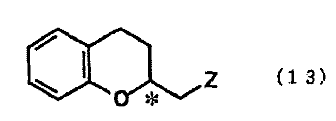 (wherein Z represents hydroxyl, alkylsulfonyloxy, arylsulfonyloxy, halogen, or amino; and the asterisked carbon atom is asymmetric).
1) EP448254 discloses a process for preparing optically active 2-hydroxymethylchroman, i.e., an optically active chroman derivative (13) with hydroxy as Z, the process including optically resolving racemic chroman-2-carboxylate by lipase-mediated hydrolysis to obtain an optically active ester and reducing the ester. Since this process basically employs an optical resolution method, the yield and the efficiency are low. Moreover, since the starting materials are not commercially available and thus need to be separately prepared, the production process is complicated and inefficient.
2) Japanese Unexamined Patent Application Publication No. 5-25158 teaches a process for producing optically active 2-hydroxymethylchroman from optically active butanetriol in seven reaction steps. This production process is long and complicated. Moreover, the optical purity of the resulting optically active 2-hydroxymethylchroman is not disclosed.
3) W099/29687 teaches a process for producing optically active 2-methanesulfonyloxymethylchroman, i.e., an optically active chroman derivative (13) with methanesulfonyloxy as Z. This process includes the steps of optically resolving racemic chroman-2-carboxylic acid by crystallizing it as optically active amine salts, esterifying and then reducing the optically active carboxylic acid to obtain optically active 2-hydoxymethylchroman, and methanesulfonylating the optically active 2-hydoxymethylchroman obtained. Since this process basically employs an optical resolution method, the yield and the efficiency are low. Moreover, since the starting materials are not commercially available and thus need to be separately prepared, the production process is complicated and inefficient.
4) EP546388 and EP546389 each teach a process for producing optically active 2-p-toluenesulfonyloxymethylchroman, i.e., an optically active chroman derivative (13) with p-toluenesulfonyloxy as Z, the process including reducing optically active chroman-2-carboxylate to obtain optically active 2-hydroxymethylchroman and p-toluenesulfonylating the optically active 2-hydroxymethylchroman. However, neither publication teaches a process for producing optically active chroman-2-carboxylate. In order to produce optically active chroman-2-carboxylate, conventional processes, such as those described in paragraphs 1) and 3), must still be employed. However, these processes are complicated, low-yield, and inefficient.
5) EP707007 teaches a process for producing optically active 2-aminomethylchroman, i.e., an optically active chroman derivative (13) with amino as Z, by twice crystallizing racemic 2-aminomethylchroman as diastereomer salts with L-N-tosylproline. However, this process employs an optical resolution method requiring crystallization twice. Thus, the yield and efficiency are low. Moreover, since the starting materials are not commercially available and thus need to be separately prepared, the production process is complicated and inefficient.
6) EP707007 also discloses a process for producing optically active 2-aminomethylchroman, the process including optically resolving racemic chroman-2-carboxylic acid as an optically active amine salt, refining by chromatography with an optical resolution column to obtain optically active chroman-2-carboxylate, and ammonolyzing and reducing the optically active chroman-2-carboxylate. However, since this process employs an optical resolution method, the yield and the efficiency are low. Moreover, this process requires an optical resolution column and is thus impractical. The starting materials are not commercially available and thus need to be separately prepared. Accordingly, the production process is complicated and inefficient.
7) US5137901 teaches a method for producing optically active 2-aminomethylchroman by preparing an amide of optically active phenethylamine from chroman-2-carboxylic acid, resolving diastereomers by crystallization, and reducing the resulting product. However, since this method also employs an optical resolution method, the yield and the efficiency are low. Moreover, since the starting materials are not commercially available and thus need to be separately prepared, the production process is complicated and inefficient.

As is described above, most conventional techniques for producing optically active chroman derivatives (13) employ low-yield, inefficient optical resolution and are thus impractical. Moreover, to produce the optically active chroman derivatives (13) from commercially available materials requires a long, complicated, and inefficient process, which is impractical. Furthermore, a process for producing an optically active 2-halomethylchroman derivative, i.e., an optically active chroman derivative (13) with halogen as Z has not been known.

### Disclosure of Invention

The present invention provides a highly practical method for producing optically active chroman derivatives. In other words, the present invention provides a practical process for efficiently producing various optically active chroman derivatives from commercially available materials in fewer steps in high yield.

The present inventors have found, through extensive investigations, a process for producing various optically active chroman derivatives from widely available, inexpensive dihydrocoumarin as the starting material, and have realized the present invention. In this process, cyclic hemiacetal derivatives prepared from dihydrocoumarin are enantioselectively reduced to prepare various optically active chroman derivatives.

In particular, the present invention relates to a method for producing a cyclic hemiacetal derivative represented by general formula (1): 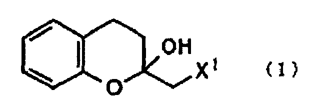 (wherein X¹ represents halogen), the method including reacting dihydrocoumarin with an enolate prepared from a base and a haloacetic acid derivative represented by general formula (2): 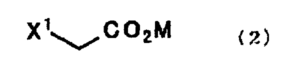 (wherein X¹ represents halogen, M represents hydrogen, alkali metal, or halogenated alkali earth metal), followed by acid treatment.

The present invention also relates to a method for producing optically active halohydrin represented by general formula (5) 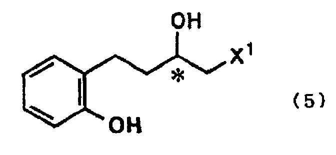 (wherein X¹ represents halogen and the asterisked carbon atom is asymmetric), the method including enantioselectively reducing one or a mixture of a cyclic hemiacetal derivative represented by formula (1): 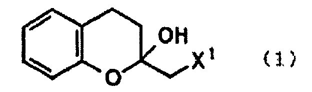 (wherein X¹ represents halogen), and a haloketone derivative represented by formula (4) 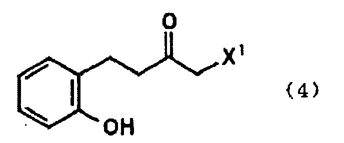 (wherein X¹ represents halogen), using a culture broth, a cellular fraction, or a processed matter of a microorganism.

The present invention provides a method for producing optically active 2-hydroxymethylchroman represented by formula (6) 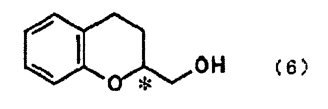 (wherein the asterisked carbon atom is asymmetric), the method including cyclizing optically active halohydrin represented by general formula (5): 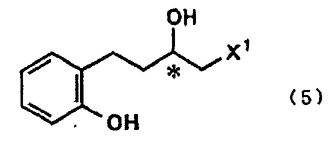 (wherein X¹ represents halogen and the asterisked carbon atom is asymmetric) using a base.

The present invention relates to a process for producing an optically active sulfonyloxymethylchroman derivative represented by general formula (7) 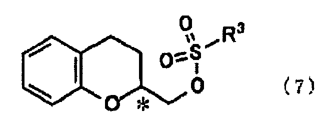 (wherein R³ represents C₁-C₁₂ alkyl or C₆-C₁₂ aryl and the asterisked carbon atom is asymmetric), the method including cyclizing optically active halohydrin represented by general formula (5): 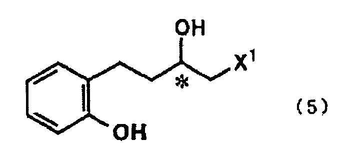 (wherein X¹ represents halogen and the asterisked carbon atom is asymmetric) using a base, and reacting the resulting product with a sulfonylating agent.

The present invention provides a method for producing optically active aminomethylchroman derivative represented by formula (9): 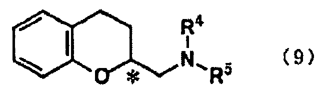 (wherein R⁴ and R⁵ each independently represent hydrogen, C₁-C₁₂ alkyl, C₁-C₂₀ aralkyl, formyl, or C₂-C₁₂ acyl, and the asterisked carbon atom is asymmetric), the method including cyclizing optically active halohydrin represented by general formula (5): 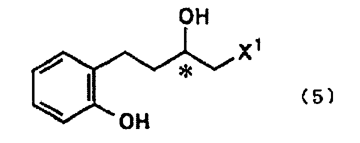 (wherein X¹ represents halogen and the asterisked carbon atom is asymmetric) using a base, subsequently reacting the resulting product with a sulfonylating agent to prepare an optically active sulfonyloxymethylchroman derivative represented by general formula (7): 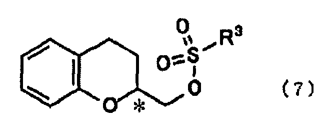 (wherein R³ represents C₁-C₁₂ alkyl or C₆-C₁₂ aryl, and the asterisked carbon atom is asymmetric), and then reacting the optically active sulfonyloxymethylchroman derivative represented by general formula (7) with a nitrogen-containing compound represented by general formula (8): 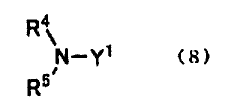 (wherein R⁴ and R⁵ are the same as above, and Y¹ represents hydrogen or alkali metal).

The present invention also relates to a method for producing optically active 2-halomethylchroman represented by general formula (11): 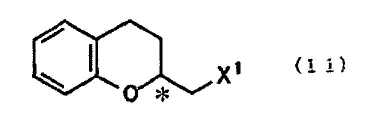 (wherein X¹ represents halogen and the asterisked carbon atom is asymmetric), the method including reacting an optically active disulfonate derivative represented by general formula (10) 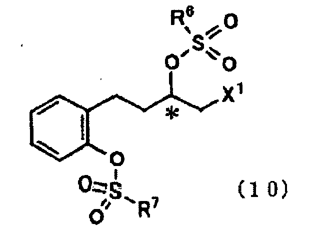 (wherein X¹ is the same as above; R⁶ and R⁷ are each independently C₁-C₁₂ alkyl or C₆-C₁₂ aryl; and * is the same as above) with a base in a protic solvent or a mixed solvent containing a protic solvent so as to perform solvolysis of sulfonyl group and cyclization.

The present invention also relates to a process for producing an optically active aminomethylchroman derivative represented by general formula (9): 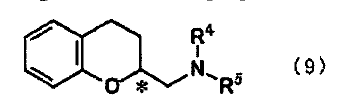 (wherein R⁴ and R⁵ each independently represent hydrogen, C₁-C₁₂ alkyl, C₁-C₂₀ aralkyl, formyl, or C₂-C₁₂ acyl, and the asterisked carbon atom is asymmetric), the method including reacting an optically active disulfonate derivative represented by general formula (10): 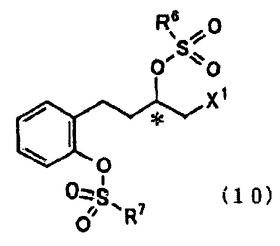 (wherein X¹ represents halogen; R⁶ and R⁷ each independently represent C₁-C₁₂ alkyl or C₆-C₁₂ aryl; and * is the same as above) with a base in a protic solvent or a mixed solvent containing a protic solvent so as to perform solvolysis of sulfonyl group and cyclization to prepare optically active 2-halomethylchroman represented by general formula (11): 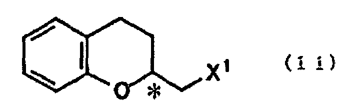 (wherein X¹ is the same as above), and reacting the optically active 2-halomethylchroman with a nitrogen-containing compound represented by general formula (8): 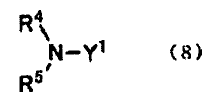 (wherein R⁴ and R⁵ are the same as above, and Y¹ represents hydrogen or alkali metal).

The present invention also relates to a cyclic hemiacetal derivative represented by general formula (1): 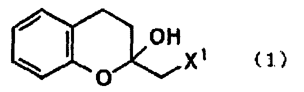 (wherein X¹ represents halogen).

The present invention also relates to an optically active halohydrin derivative represented by general formula (3): 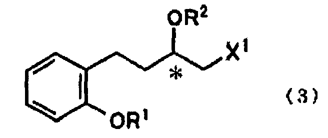 (wherein X¹ represents halogen; R¹ and R² each independently represent hydrogen, C₁-C₁₂ alkylsulfonyl, C₆-C₁₂ arylsulfonyl, C₁-C₁₀ acyl, or C₃-C₁₂ silyl; and the asterisked carbon atom is asymmetric).

The present invention also relates to an (R)-2-halomethylchroman derivative represented by general formula (12): 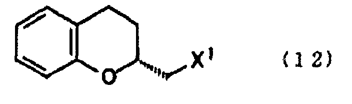 (wherein X¹ represents halogen).

### Best Mode for Carrying Out the Invention

The present invention can be briefly described as in Scheme 1 below. 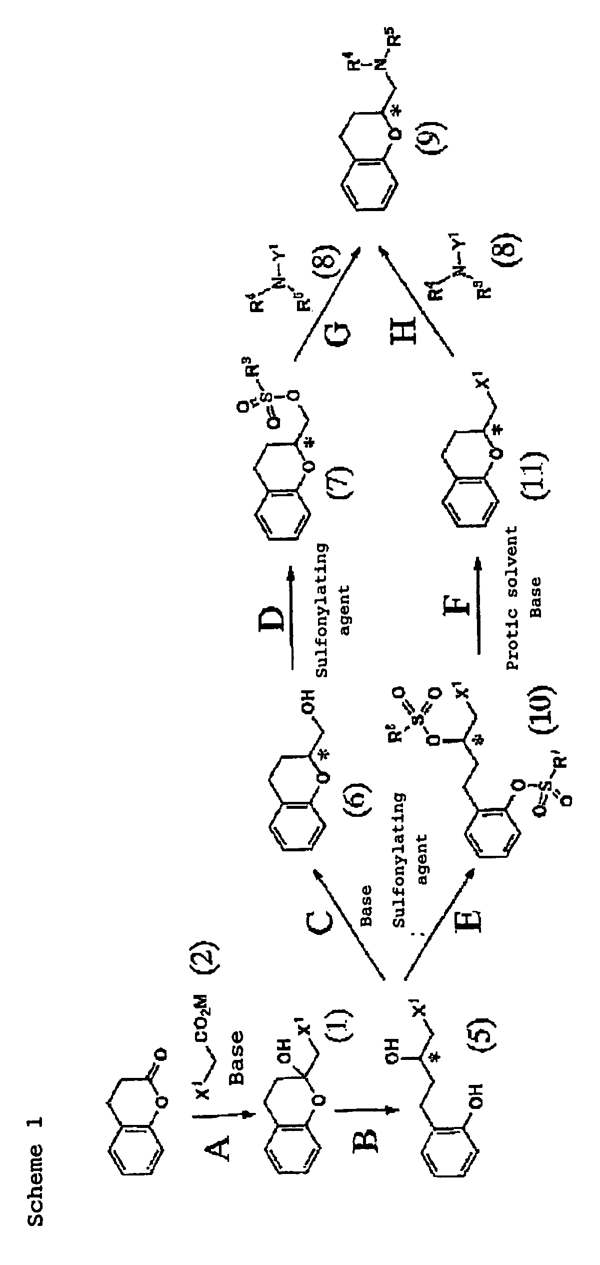
Step A: An enolate prepared from a haloacetic acid derivative (2) and a base is reacted with dihydrocoumarin, followed by acid treatment, to synthesize a cyclic hemiacetal derivative (1).
Step B: The cyclic hemiacetal derivative (1) is enantioselectively reduced to prepare optically active halohydrin (5).
Step C: The optically active halohydrin (5) is cyclized with a base to prepare optically active 2-hydroxymethylchroman (6).
Step D: The optically active 2-hydroxymethylchroman (6) is reacted with a sulfonylating agent to synthesize an optically active sulfonyloxymethylchroman derivative (7).
Step E: The optically active halohydrin derivative (5) is reacted with a sulfonylating agent to prepare an optically active disulfonate derivative (10).
Step F: The optically active disulfonate derivative (10) is reacted with a base in a protic solvent to prepare optically active 2-halomethylchroman (11).
Step G: The optically active sulfonyloxymethylchroman derivative (7) is reacted with a nitrogen-containing compound (8) to prepare an optically active aminomethylchroman derivative (9).
Step H: The optically active 2-halomethylchroman (11) is reacted with the nitrogen-containing compound (8) to prepare an optically active aminomethylchroman derivative (9).

As shown in Scheme 1 above, optically active chroman derivatives represented by general formula (13): 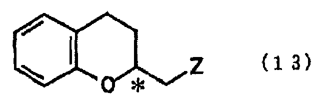 (wherein Z represents hydroxyl, alkylsulfonyloxy, arylsulfonyloxy, halogen, or amino, and the asterisked carbon atom is asymmetric) are produced from inexpensive, commercially available starting materials in three to five steps by suitably combining Steps A to H. Since the present invention uses enantioselective reactions instead of optical resolution methods to introduce asymmetry, optically active materials can be produced in high yield.

The resulting optically active chroman derivatives are useful as pharmaceutical intermediates, in particular, synthetic intermediates for serotonin receptor agonists. Serotonin receptor agonists are promising therapeutic drugs for the central nervous system, e.g., drugs for motor disorders.

Each step of the present invention will now be described in detail.

### Step A

In this step, a haloacetic acid derivative (2): 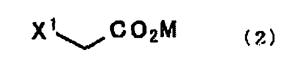 is reacted with a base to prepare an enolate. The enolate is reacted with dihydrocoumarin, followed by acid treatment, to prepare a cyclic hemiacetal derivative (1):

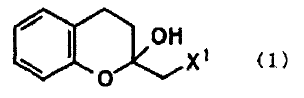

In the haloacetic acid derivative (2), X¹ represents halogen. Examples of the halogen include fluorine, chlorine, bromine, and iodine. In view of the availability of the starting material, X¹ is preferably chlorine or bromine, and more preferably chlorine.

M represents hydrogen, alkali metal, or halogenated alkali earth metal. Examples of M are not limited to but include hydrogen, lithium, sodium, potassium, magnesium chloride, magnesium bromide, and calcium chloride. In view of the availability of the starting material, M is preferably sodium or magnesium chloride, and more preferably sodium.

Accordingly, preferred examples of the haloacetic acid derivative (2) include sodium chloroacetate, sodium bromoacetate, magnesium chloride chloroacetate, and magnesium bromide chloroacetate.

Magnesium chloride chloroacetate prepared in the reaction system by mixing sodium chloroacetate and magnesium chloride and depositing sodium chloride may also be used.

The amount of the haloacetate derivative (2) used is 1 to 10 molar equivalents, and preferably 1 to 3 molar equivalents relative to dihydrocoumarin. At an amount of the haloacetate derivative (2) less than 1 molar equivalent, the reaction yield tends to decrease. An amount of the haloacetate derivative (2) exceeding 10 molar equivalents is economically disadvantageous.

The base used to prepare enolate from the haloacetate derivative (2) is not particularly limited. Examples of the base include metal amides such as lithium amide, sodium amide, lithium diisopropylamide, magnesium chloride diisopropylamide, magnesium bromide diisopropylamide, and magnesium chloride dicyclohexylamide; alkyl metals such as methyllithium, n-butyllithium, methylmagnesium bromide, i-propylmagnesium chloride, and tert-butylmagnesium chloride; metal alkoxides such as sodium methoxide, magnesium ethoxide, and potassium tert-butoxide; and metal hydrides such as lithium hydride, sodium hydride, potassium hydride, and calcium hydride. In particular, tert-butylmagnesium chloride is preferred because it achieves high yield.

Preferably, 1 to 10, and more preferably 2 to 6 molar equivalents of the base to dihydrocoumarin is used. At an amount less than 1 molar equivalent, the reaction yield tends to decrease. An amount exceeding 10 molar equivalents is economically disadvantageous.

In this process, the yield sometimes increases in the presence of amines during the reaction of enolate and dihydrocoumarin.

The amine is preferably a tertiary amine. Examples thereof include alkyl amines such as triethylamine, tributylamine, diisopropylethylamine, trioctylamine, N-methylmorpholine, N-methylpyrrolidine, and N-methylpiperidine; aryl amines such as dimethylaniline and diethylaniline; and aromatic amines such as pyridine, quinoline, and isoquinoline. Triethylamine is particularly preferred due to its easy handling and wide availability.

The amount of the amine used is preferably 1 to 5 molar equivalents to dihydrocoumarin but may be larger. With less than 1 molar equivalent of the amine, the effect of adding amine diminishes, which is not desirable.

In this process, the order of adding the haloacetic acid derivative, the base, dihydrocoumarin, and the amine may be any suitable order. For example, preparation of the enolate and reaction of the enolate with dihydrocoumarin can be performed at the same time by adding a base solution dropwise into a mixed solution of dihydrocoumarin, a halogenated acetic acid derivative, and amine.

In this process, the reaction solvent is not particularly limited but is preferably a aprotic organic solvent when a metal amide or alkyl metal is used as the base. Examples of the aprotic organic solvent include hydrocarbon solvents such as benzene, toluene, n-hexane, and cyclohexane; ether solvents such as diethylether, tetrahydrofuran (THF), 1,4-dioxane, methyl t-butyl ether, dimethoxyethane, and ethylene glycol dimethylether; halogenated solvents such as methylene chloride, chloroform, and 1,1,1,-trichloroethane; and aprotic polar solvents such as dimethylformamide, N-methylpyrrolidone, and hexamethylphosphoric triamide. These may be used alone or in combination. Ether solvents such as THF are particularly preferred since they well dissolve the materials.

The reaction temperature is typically about -100°C to about 120°C. A preferable reaction temperature differs depending on the type of base used. When tert-butylmagnesium chloride is used as the base, the reaction temperature is preferably -20°C to 60°C to increase the yield.

The acid used in the acid treatment of this process may be a common organic or inorganic acid. Examples of the acid include hydrochloric acid, sulfuric acid, nitric acid, acetic acid, and citric acid. The temperature of acid treatment is not particularly limited but is preferably -20 to 60°C.

In this process, the completion of the reaction is determined by confirming the absence of dihydrocoumarin through analysis such as thin-layer chromatography, highperformance liquid chromatography, or gas chromatography.

Upon completion of a series of reactions, a typical work up may be carried out to isolate the products from the reaction mixture. For example, upon completion of the acid treatment, the product is extracted with a typical extraction solvent such as ethyl acetate, diethyl ether, methylene chloride, toluene, or hexane. During the extraction, alkali washing may be performed to remove the remaining haloacetic acid derivative (2).

The extract is, for example, heated under a reduced pressure to remove the reaction solvent and the extraction solvent to obtain the target product. The target product is nearly pure but may be purified through a typical process, such as crystallization, fractional distillation, or column chromatography, to increase the purity.

### Step B

In Step B of the present invention, the present inventors have developed a reducing process that uses a microorganism to produce an optically active halohydrin represented by general formula (5): 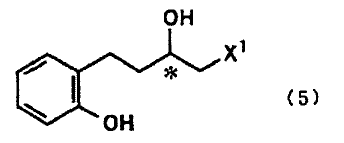 through enantioselectively reducing a cyclic hemiacetal derivative (1): 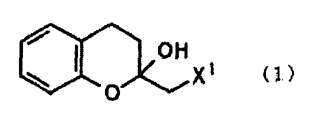 or a haloketone derivative (4):

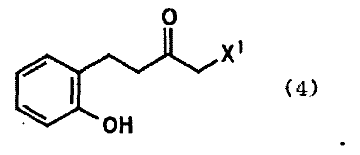

In particular, the optically active halohydrin represented by general formula (5) 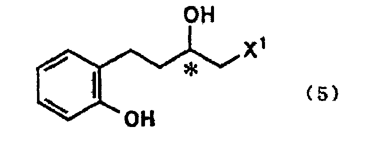 is prepared by enantioselectively reducing a cyclic hemiacetal derivative represented by general formula (1) 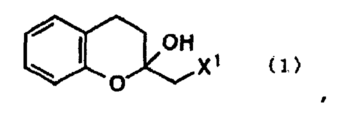 a haloketone derivative represented by general formula (4) 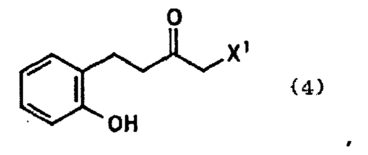 or a mixture of these while using a culture broth, a cellular fraction, or a processed matter of a microorganism.

The cyclic hemiacetal derivative (1) and the haloketone derivative (4) are ketonic isomers of intermolecular hemiacetal. In a solution, the cyclic hemiacetal derivative (1) spontaneously transforms into the haloketone derivative (4), while the haloketone derivative (4) spontaneously transforms into the cyclic hemiacetal derivative (1). Since the transformations reach an equilibrium under particular conditions, the ratio of the two derivatives is always constant in the solution. Accordingly, both the cyclic hemiacetal derivative (1) and the haloketone derivative (4) can be used as the starting materials of Step B of preparing optically active halohydrin (5) through enantioselective reduction. A mixture of the cyclic hemiacetal derivative (1) and the haloketone derivative (4) may also be used as the starting material of Step B.

X¹ of the cyclic hemiacetal derivative (1) and the haloketone derivative (4), i.e., the starting materials of Step B, represents halogen. X¹ is preferably chlorine since the raw material therefor is easy to obtain.

In the formula of an optically active halohydrin (5), the asterisked carbon atom is asymmetric. The configuration of the asymmetric carbon in the optically active halohydrin (5) obtained in this step is either (R) or (S). In general, for producing the optically active halohydrin (5) useful as the intermediate for serotonin receptor agonists, the asymmetric carbon preferably has the (S) configuration since pharmaceutical intermediates described in European Patent No. 707007 can be derived, for example.

The microorganism that transforms the cyclic hemiacetal derivative (1) or the haloketone derivative (4) into optically active halohydrin (5) through enantioselective reduction can be obtained according to the method described below.

First, a cellular fraction is harvested from 5 ml of a culture broth of a microorganism by centrifugation or the like. The cellular fraction is suspended in 2.5 ml of a 100 mM phosphate buffer solution (pH 6.5) containing 1.25 to 2.5 mg of 1-chloromethyl-1-hydroxychroman and 125 mg of glucose. The suspension is added to a test tube and shaken at 30°C for 2 to 3 days. After the conversion reaction, ethyl acetate is added to the reaction mixture to perform extraction. 1-Chloro-4-(2-hydroxyphenyl)butan-2-ol in the resulting extract is analyzed by highperformance liquid chromatography (column: Daicel Chiralcel OD-H (trademark) (4.6 mm x 250 mm); eluent: n-hexane/isopropanol = 9/1; flow rate: 0.8 ml/min; detection: 210 nm; column temperature: 30°C; elution time ((2S)-1-chloro-4-(2-hydroxyphenyl)butan-2-ol: 17.1 min, (2R)-1-chloro-4-(2-hydroxyphenyl)butan-2-ol: 45.35 min)) to evaluate the reducing capacity.

Among the microorganisms selected by the above-described analysis in the present invention, microorganisms belonging to the genus Candida are preferred. Those belonging to the species Candida magnoliae and Candida maris are more preferred, and Candida magnoliae IF0705 and Candida maris IFO10003 are most preferred. These microorganisms can be obtained from stock strains readily available or purchasable or can be isolated from the natural world. It is also possible to obtain strains having favorable properties for this reaction by causing mutation of these microorganisms.

In culturing these microorganisms, any of the media containing nutrient sources assimilable by these microorganisms can generally be used. For example, a medium may contain a carbon source, for example, saccharides, such as glucose, sucrose, and maltose; organic acids, such as lactic acid, acetic acid, citric acid, and propionic acid; alcohols, such as ethanol and glycerin; hydrocarbons, such as paraffin; fats and oils, such as soybean oil and rapeseed oil; and a mixture of these. The medium may also contain a nitrogen source, for example, ammonium sulfate, ammonium phosphate, urea, yeast extracts, meat extracts, peptone, and corn steep liquor. The medium may further contain other nutrients such as inorganic salts and vitamins, if necessary.

The culture of the microorganism may be carried out under ordinary conditions, e.g., a pH of 4.0 to 9.5 and a temperature in the range of 20°C to 45°C, for 10 to 96 hours under aerobic conditions.

In reacting the enzyme derived from the microorganism with the cyclic hemiacetal derivative (1) and the haloketone derivative (4), a culture broth containing the microorganism can be directly used. When the culture broth contains a component that adversely affects the reaction, a cellular fraction obtained by centrifugation of the culture broth or a processed matter of a microorganism is preferably used.

Examples of the processed matter are not particularly limited but include dry bacteria cells prepared by dehydration with acetone or diphosphorus pentaoxide or by drying using a desiccator or an electric fan; materials derived by surfactant treatment; materials derived by lytic enzyme treatment; immobilized cells; and cell-free extract preparations derived by disruption of cells. It is also possible to use an enzyme catalyzing the asymmetric reduction derived from the culture by purification.

Microorganisms derived by genetic techniques, such as cell fusion or recombinant DNA technique, may also be used as the enzyme source described above. Microorganisms, which are transformed to produce enzymes that catalyze the enantioselective reaction, can be produced by a method including the steps of separating/purifying an enzyme that catalyzes an enantioselective reduction to determine all or part of the amino acid sequences of the enzyme; obtaining DNA sequences that code the enzyme based on the amino acid sequences determined; introducing the DNAs to other microorganisms to obtain genetically modified microorganisms; and culturing the genetically modified microorganisms to obtain the enzyme (WO 98/35025, WO 01/00599).

In carrying out the reduction, a substrate, i.e., the cyclic hemiacetal derivative (1) or the haloketone derivative (4), may be added all at once at the beginning of the reaction or in divided portions with the progress of the reaction.

The temperature during the reaction is normally 10 to 60°C, and preferably 20 to 40°C. The pH during the reaction is 2.5 to 9, and preferably 5 to 9.

The concentration of the microorganism in the reaction mixture may be selected according to its ability to reduce the substrate. The substrate concentration in the reaction mixture is preferably 0.01 to 50% and more preferably 0.1 to 30% (W/V). At a substrate concentration less than 0.01%, the productivity becomes markedly low. At a substrate concentration exceeding 50%, the reaction yield tends to decrease.

The reaction is generally carried out with shaking or with aeration and stirring to promote the reaction and increase the yield. The reaction time is adequately selected based on the substrate concentration, the microorganism concentration, and other reaction conditions. For increasing the productivity, the reaction conditions are preferably selected so that the reaction is completed in 2 to 168 hours.

In order to accelerate the reduction, 1 to 30% (W/V) of an energy source, such as glucose or ethanol, is preferably added to the reaction mixture to obtain better results. The addition of a coenzyme, such as nicotinamide adenine dinucleotide (reduced form, NADH) or nicotinamide adenine dinucleotide phosphate (reduced form, NADPH), necessary for biological reduction can also accelerate the reaction. The coenzyme may be directly added to the reaction mixture. Alternatively, a reaction system that generates NADH or NADPH and an oxidized form of the coenzyme (NAD or NADP) may be added to the reaction mixture. For example, a reaction system that reduces NAD into NADH during the course of producing water and carbon dioxide from formic acid using a formate dehydrogenase, or a reaction system that can reduce NAD into NADH or NADP into NADPH during the course of producing gluconolactone from glucose using a glucose dehydrogenase may be employed.

A culture or a processed product of a transformed microorganism produced by introducing a gene for an enzyme that catalyzes enantioselective reduction and a gene for an enzyme capable of regenerating a coenzyme NAD(P)H, upon which the enzyme that catalyzes enantioselective reduction depends, is preferably used in the reaction described above. According to this process, an optically active halohydrin (5) can be produced without adding an additional enzyme necessary for regenerating the reduced coenzyme.

Examples of the transformed microorganism include transformed cells, which are transformed using a plasmid having a DNA that codes an enzyme that catalyzes enantioselective reduction (e.g., an enzyme derived from a microorganism of the genus Candida) and a DNA that codes an enzyme capable of regenerating the coenzyme upon which the enzyme that catalyzes enantioselective reduction depends. Preferred examples of the transformed cells include transformed cells in which the enzyme capable of regenerating the coenzyme is a glucose dehydrogenase; transformed cells in which the glucose dehydrogenase is derived from Bacillus megaterium; transformed cells in which the plasmid is pNTS1G or pNTFPG; and Escherichia coli. More preferable examples of the transformed cell are Escherichia coli HB101 (pNTS1G), accession number FERM BP-5835 (deposited with International Patent Organism Depositary, National Institute of Advanced Industrial Science and Technology, Tsukuba Central 6, 1-1-1 Higashi, Tsukuba, Ibaraki, Japan, since February 24, 1997) and Escherichia coli HB101 (pNTFPG), accession number FERM BP-7117 (deposited with International Patent Organism Depositary, National Institute of Advanced Industrial Science and Technology, Tsukuba Central 6, 1-1-1 Higashi, Tsukuba, Ibaraki, Japan, since April 11, 2000).

It is also effective to add a surfactant, such as Triton (product Nacalai Tesque Inc.), Span (product of Kanto Kagaku), or Tween (product of Nacalai Tesque Inc.), into the reaction mixture. A water-insoluble organic solvent, such as ethyl acetate, butyl acetate, isopropylether, or toluene, may be added to the reaction mixture for the purpose of avoiding the inhibition of the reaction by the substrate and/or alcohols produced by the reduction. For increasing the solubility of the substrate, a water-soluble organic solvent, such as methanol, ethanol, acetone, tetrahydrofuran, or dimethylsulfoxide, may also be added.

In this step, the completion of the reaction is detected by confirming the absence of the starting materials through analysis such as thin-layer chromatography, highperformance liquid chromatography, or gas chromatography.

The optically active halohydrin (5) produced by the reduction is recovered directly from the reaction mixture. Alternatively, optically active halohydrin (5) may be recovered by removing a cellular fraction and the like, extracting optically active halohydrin (5) with a solvent such as ethyl acetate or toluene, and removing the solvent. The obtained optically active halohydrin (5) may be purified by recrystallization, silica gel column chromatography, or the like to obtain a high-purity optically active halohydrin (5).

### Step C

In this step, optically active halohydrin (5): 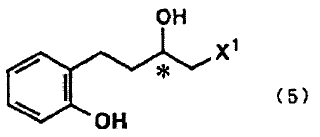 is cyclized with a base to prepare optically active 2-hydroxymethylchroman (6):

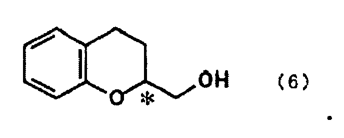

Optically active halohydrin (5) used in this step is, for example, the optically active halohydrin (5) prepared in Step B. Thus, X¹ is halogen and is preferably chlorine.

The base used in this step is not particularly limited. Examples of the base include metal hydroxides such as sodium hydroxide, potassium hydroxide, lithium hydroxide, magnesium hydroxide, and barium hydroxide; carbonates such as sodium carbonate, potassium carbonate, and sodium hydrogen carbonate; metal amides such as lithium amide, sodium amide, lithium diisopropylamide, magnesium chloride diisopropylamide, magnesium bromide diisopropylamide, and magnesium chloride dicyclohexylamide; alkyl metals such as methyllithium, n-butyllithium, methylmagnesium bromide, i-propylmagnesium chloride, and tert-butylmagnesium chloride; metal alkoxides such as sodium methoxide, magnesium ethoxide, and potassium tert-butoxide; metal hydrides such as lithium hydride, sodium hydride, potassium hydride, and calcium hydride; and amines such as triethylamine, diisopropylethylamine, N-methylmorphorine, dimethylaniline, and pyridine. Sodium hydride and metal hydroxides, such as sodium hydroxide, potassium hydroxide, and lithium hydroxide, are inexpensive and are thus preferred as the base used in this step. Preferably, 1 to 10 molar equivalents of the base is used.

The reaction solvent used in this step is not particularly limited but is preferably a protic solvent or an aprotic solvent. Examples of the protic solvent include water and alcohol solvents such as methanol, ethanol, isopropanol, and n-butanol. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, and 1,1,1,-trichloroethane; and aprotic polar solvents such as dimethylformamide, N-methylpyrrolidone, and hexamethylphosphoric triamide. These solvents may be used alone or in combination.

A suitable solvent is selected according to the properties of the base used. When a metal hydroxide is used as the base, water is preferably used as the solvent or as one component of a mixed solvent to increase the reaction yield.

As is described below in relation to Step D, Step D can be successively performed without a work up of Step C to further effectively implement the present invention. In such a case, the solvent is preferably THF.

The reaction temperature of this step is in the range of -80 to 100°C. Preferably, the reaction temperature is in the range of 0 to 50°C to increase the yield. At a reaction temperature less than -80°C, the yield decreases. At a reaction temperature exceeding 100°C, side reaction may occur.

In this step, the completion of the reaction is detected by confirming the absence of optically active halohydrin (5) by analysis such as thin-layer chromatography, highperformance liquid chromatography, or gas chromatography.

In order to isolate optically active 2-hydroxymethylchroman (6) synthesized through Step C, a typical work up may be carried out. For example, the base may be neutralized with a common inorganic acid such as hydrochloric acid or sulfuric acid, followed by extraction using a common extraction solvent, such as ethyl acetate, diethylether, methylene chloride, toluene, or hexane.

The obtained extract is, for example, heated under a reduced pressure to remove the reaction solvent and the extraction solvent to obtain the target substance. The target substance obtained is nearly pure but may be purified by crystallization, fractional distillation, or column chromatography, to increase the purity.

In the formulae representing optically active halohydrin (5) and optically active 2-hydroxymethylchroman (6), the asterisked carbon atom is asymmetric.

In the course of the reaction of this step, the configuration of the asymmetric carbon becomes inverted. In particular, (R) isomers of optically active 2-hydroxymethylchroman (6) are obtained from (S) isomers of optically active halohydrin (5).

In general, in producing optically active 2-hydroxymethylchroman (6) for use as intermediates for serotonin receptor agonists, (R) isomers of 2-hydroxymethylchroman are preferably prepared from (S) isomers of optically active halohydrin (5). In this manner, pharmaceutical intermediates disclosed in European Patent No. 707007 can be derived, for example.

### Step D

In this step, an optically active sulfonyloxymethylchroman derivative (7): 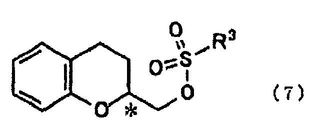 is prepared by reacting optically active 2-hydroxymethylchroman (6): 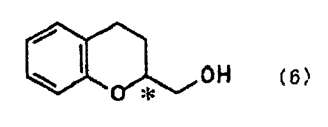 with a sulfonylating agent.

The sulfonylating agent used in this step is not particularly limited. Any suitable commercially available sulfonylating agent may be used. Examples of the sulfonylating agent include aliphatic sulfonyl chlorides, aromatic sulfonyl chlorides, aliphatic sulfonic anhydrides, and aromatic sulfonic anhydrides. Specific examples of the sulfonylating agent include methanesulfonyl chloride, trifluoromethanesulfonyl chloride, benzenesulfonyl chloride, p-toluenesulfonyl chloride, nitrobenzenesulfonyl chloride, methanesulfonic anhydride, trifluoromethanesulfonic anhydride, benzenesulfonic anhydride, p-toluenesulfonic anhydride, and nitrobenzenesulfonic anhydride.

Among these, methanesulfonyl chloride, benzenesulfonyl chloride, p-toluenesulfonyl chloride, and nitrobenzenesulfonyl chloride are preferred for their wide availability. Methanesulfonyl chloride is particularly preferred since it is inexpensive.

Accordingly, in the optically active sulfonyloxymethylchroman derivative (7) prepared in this step, R³ represents C₁-C₁₂ alkyl or C₆-C₁₂ aryl, in particular, methyl, ethyl, octyl, decyl, dodecyl, trifluoromethyl, phenyl, tolyl, nitrophenyl, methoxyphenyl, or naphthyl. Methyl, phenyl, p-tolyl, and nitrophenyl are preferred since they are inexpensive and widely available.

A tertiary amine may be used to accelerate the reaction with the sulfonylating agent since the tertiary amine functions as a reaction catalyst. The tertiary amine used is not particularly limited. Examples of the tertiary amine include alkylamines such as triethylamine, tributylamine, diisopropylethylamine, trioctylamine, N-methylmorphorine, N-methylpyrrolidine, and N-methylpiperidine; arylamines such as dimethylaniline and diethylaniline; and aromatic amines such as pyridine, quinoline, and isoquinoline. Triethylamine is preferred for its easy handling and wide availability.

The reaction solvent used in this step is not particularly limited but is preferably a protic solvent or an aprotic solvent. Examples of the protic solvent include water and alcohol solvents such as methanol, ethanol, isopropanol, and n-butanol. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, 1,1,1,-trichloroethane; and aprotic polar solvents such as dimethylformamide, N-methylpyrrolidone, and hexamethylphosphoric triamide. These solvents may be used alone or in combination.

In order to increase the efficiency, Step C and Step D of the present invention may be successively carried out. That is, the sulfonylating agent may be successively added without the work up of Step C after completion of the reaction so as to facilitate the production of the optically active sulfonyloxymethylchroman derivative (7). When no work up is performed in Step C, the same reaction solvent can be used in Steps C and D. Here, the reaction solvent is preferably THF.

The reaction temperature of this step is in the range of -80 to 150°C. Preferably, the reaction temperature is in the range of 0 to 100°C to increase the yield.

The completion of the reaction of this step is detected by confirming the absence of optically active 2-hydroxymethylchroman (6) by analysis, such as thin-layer chromatography, highperformance liquid chromatography, or gas chromatography.

In order to isolate the optically active sulfonyloxymethylchroman derivative (7) produced in this step, a typical work up may be carried out. For example, water, diluted hydrochloric acid, or diluted alkali may be added to terminate the reaction, followed by extraction using a typical extraction solvent, such as ethyl acetate, diethylether, methylene chloride, toluene, or hexane.

The obtained extract is, for example, heated under a reduced pressure to remove the reaction solvent and the extraction solvent to obtain the target substance. The target substance obtained is nearly pure but may be purified by recrystallization, fractional distillation, or column chromatography, to increase the purity.

In the formulae representing optically active 2-hydroxymethylchroman (6) and the optically active sulfonyloxymethylchroman derivative (7), the asterisked carbon atom is asymmetric.

The reaction of this step progresses with retention of the configuration at the asymmetric carbon. In particular, (R) isomers of the optically active sulfonyloxymethylchroman derivative (7) are obtained from (R) isomers of optically active 2-hydroxymethylchroman (6).

In general, in producing the optically active sulfonyloxymethylchroman derivative (7) as the intermediates for serotonin receptor agonists, the (R) isomers of the optically active sulfonyloxymethylchroman derivative (7) are preferably formed from (R) isomers of optically active 2-hydroxymethylchroman. In this manner, pharmaceutical intermediates disclosed in European Patent No. 707007 can be derived, for example.

### Step E

In this step, an optically active halohydrin (5) 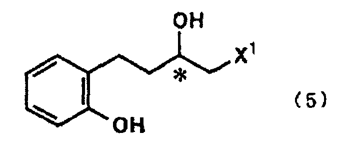 is reacted with a sulfonylating agent to produce an optically active disulfonate derivative (10):

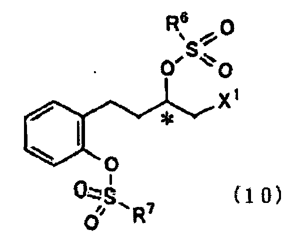

The optically active halohydrin (5) used in this step is, for example, the optically active halohydrin (5) prepared in Step B. Thus, X¹ is halogen and is preferably chlorine.

The sulfonylating agent used in this step is not particularly limited. Any suitable sulfonylating agent that is commercially available may be used. Examples of the sulfonylating agent include aliphatic sulfonyl chlorides, aromatic sulfonyl chlorides, aliphatic sulfonic anhydrides, and aromatic sulfonic anhydrides. Specific examples of the sulfonylating agent include methanesulfonyl chloride, trifluoromethanesulfonyl chloride, benzenesulfonyl chloride, p-toluenesulfonyl chloride, nitrobenzenesulfonyl chloride, methanesulfonic anhydride, trifluoromethanesulfonic anhydride, benzenesulfonic anhydride, p-toluenesulfonic anhydride, and nitrobenzenesulfonic anhydride.

Among these, methanesulfonyl chloride, benzenesulfonyl chloride, p-toluenesulfonyl chloride, and nitrobenzenesulfonyl chloride are preferred for their wide availability. Methanesulfonyl chloride is particularly preferred since it is inexpensive.

Accordingly, in the optically active disulfonate derivative (10) prepared in this step, R⁶ and R⁷ each independently represent C₁-C₁₂ alkyl or C₆-C₁₂ aryl, in particular, methyl, ethyl, octyl, decyl, dodecyl, trifluoromethyl, phenyl, tolyl, nitrophenyl, methoxyphenyl, or naphthyl. Methyl, phenyl, p-tolyl, and nitrophenyl are preferred since they are inexpensive and widely available.

A tertiary amine may be used to accelerate the reaction with the sulfonylating agent since the tertiary amine functions as the reaction catalyst. The tertiary amine is not particularly limited. Examples of the tertiary amine include alkylamines such as triethylamine, tributylamine, diisopropylethylamine, trioctylamine, N-methylmorphorine, N-methylpyrrolidine, and N-methylpiperidine; arylamines such as dimethylaniline and diethylaniline; and aromatic amines such as pyridine, quinoline, and isoquinoline. Triethylamine is preferred for its easy handling and wide availability.

The reaction solvent used in this step is not particularly limited but is preferably a protic solvent or an aprotic solvent. Examples of the protic solvent include water and alcohol solvents such as methanol, ethanol, isopropanol, and n-butanol. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, 1,1,1,-trichloroethane; and aprotic polar solvents such as dimethylformamide, N-methylpyrrolidone, and hexamethylphosphoric triamide. These solvents may be used alone or in combination.

The reaction temperature of this step is in the range of -80 to 150°C. Preferably, the reaction temperature is in the range of 0 to 100°C to increase the yield.

The completion of the reaction of this step is detected by confirming the absence of optically active halohydrin (5) by analysis, such as thin-layer chromatography, highperformance liquid chromatography, or gas chromatography.

In order to isolate the optically active disulfonate derivative (10) produced in this step, a typical work up may be carried out. For example, water, diluted hydrochloric acid, or diluted alkali may be added to terminate the reaction, followed by extraction using a typical extraction solvent, such as ethyl acetate, diethylether, methylene chloride, toluene, or hexane.

The extract obtained is, for example, heated under a reduced pressure to remove the reaction solvent and the extraction solvent to obtain the target substance. The target substance obtained is nearly pure but may be purified by crystallization, fractional distillation, or column chromatography, to increase the purity.

In the formulae representing optically active halohydrin (5) and the optically active disulfonate derivative (10), the asterisked carbon atom is asymmetric.

The reaction of this step progresses with retention of the configuration at the asymmetric carbon. In particular, (S) isomers of the optically active disulfonate derivative (10) are obtained from (S) isomers of optically active halohydrin (5). In general, in producing the optically active sulfonyloxymethylchroman derivative (7) as the intermediates for serotonin receptor agonists, the (S) isomers of the optically active disulfonate derivative (10) are preferably formed from (S) isomers of optically active halohydrin derivative. In this manner, pharmaceutical intermediates disclosed in European Patent No. 707007 can be derived, for example.

### Step F

In this step, an optically active disulfonate derivative (10) 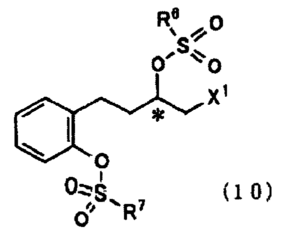 is reacted with a base in a protic solvent to perform solvolysis of sulfonyl group and cyclization so as to prepare optically active 2-halomethylchroman (11):

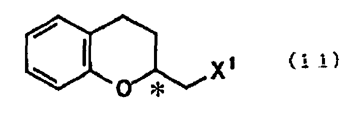

The optically active disulfonate derivative (10) used in this step is, for example the optically active disulfonate derivative (10) prepared in Step E. Thus, X¹ is halogen and is preferably chlorine. R⁶ and R⁷ each independently represent C₁-C₁₂ alkyl or C₆-C₁₂ aryl, in particular, methyl, ethyl, octyl, decyl, dodecyl, trifluoromethyl, phenyl, tolyl, nitrophenyl, methoxyphenyl, or naphthyl. Methyl, phenyl, p-tolyl, and nitrophenyl are preferred since they are inexpensive and widely available.

The base used in this step is not particularly limited. Examples of the base include metal hydroxides such as sodium hydroxide, potassium hydroxide, lithium hydroxide, magnesium hydroxide, and barium hydroxide; carbonates such as sodium carbonate, potassium carbonate, and sodium hydrogen carbonate; metal amides such as lithium amide, sodium amide, lithium diisopropylamide, magnesium chloride diisopropylamide, magnesium bromide diisopropylamide, and magnesium chloride dicyclohexylamide; alkyl metals such as methyllithium, n-butyllithium, methylmagnesium bromide, i-propylmagnesium chloride, and tert-butylmagnesium chloride; metal alkoxides such as sodium methoxide, magnesium ethoxide, and potassium tert-butoxide; metal hydrides such as lithium hydride, sodium hydride, potassium hydride, and calcium hydride; and amines such as triethylamine, diisopropylethylamine, N-methylmorphorine, dimethylaniline, and pyridine. Sodium hydride and metal hydroxides, such as sodium hydroxide, potassium hydroxide, and lithium hydroxide, are inexpensive and are thus preferred as the base used in this step. Preferably, 1 to 10 molar equivalents of the base is used.

The reaction solvent used in this step is either a protic solvent or a mixed solvent containing a protic solvent. Examples of the protic solvent include water and alcohol solvents such as methanol, ethanol, isopropanol, and n-butanol. Inexpensive water and methanol are preferred as the solvent in this step.

The mixed solvent may contain an aprotic solvent. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, and 1,1,1,-trichloroethane; and aprotic polar solvents such as dimethylformamide, N-methylpyrrolidone, and hexamethylphosphoric triamide. These solvents may be used alone or in combination.

The reaction temperature of this step is in the range of -80 to 100°C. Preferably, the reaction temperature is in the range of 0 to 80°C to increase the yield.

In this step, the completion of the reaction is detected by confirming the absence of optically active disulfonate derivative (10) by analysis such as thin-layer chromatography, highperformance liquid chromatography, or gas chromatography.

In order to isolate optically active 2-halomethylchroman (11) synthesized in this step, a typical work up may be carried out. For example, the base may be neutralized with a common inorganic acid such as hydrochloric acid or sulfuric acid, followed by extraction using a common extraction solvent, such as ethyl acetate, diethylether, methylene chloride, toluene, or hexane.

The obtained extract is, for example, heated under a reduced pressure to remove the reaction solvent and the extraction solvent to obtain the target substance. The target substance obtained is nearly pure but may be purified by crystallization, fractional distillation, or column chromatography, to increase the purity.

In the formulae representing the optically active disulfonate derivative (10) and optically active 2-halomethylchroman (11), the asterisked carbon atom is asymmetric.

In the course of the reaction of this step, the configuration of the asymmetric carbon becomes inverted. In particular, (R) isomers of optically active 2-halomethylchroman (11) are obtained from (S) isomers of optically active disulfonate derivative (10). In general, in producing optically active 2-halomethylchroman (11) for use as intermediates for serotonin receptor agonists, (R) isomers of optically active 2-halomethylchroman (11) are preferably prepared from (S) isomers of optically active disulfonate derivative (10). In this manner, pharmaceutical intermediates disclosed in European Patent No. 707007 can be derived, for example.

### Step G

An optically active sulfonyloxymethylchroman derivative (7): 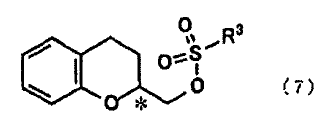 is reacted with a nitrogen-containing compound (8): 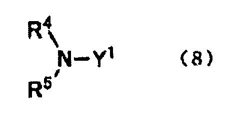 to produce an optically active aminomethylchroman derivative (9):

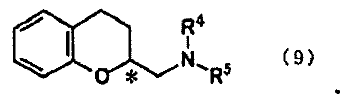

The optically active sulfonyloxymethylchroman derivative (7) used in this step is, for example, the same optically active sulfonyloxymethylchroman derivative (7) prepared in Step D. Thus, R³ represents C₁-C₁₂ alkyl or C₆-C₁₂ aryl, in particular, methyl, ethyl, octyl, decyl, dodecyl, trifluoromethyl, phenyl, tolyl, nitrophenyl, methoxyphenyl, or naphthyl. Methyl, phenyl, p-tolyl, and nitrophenyl are preferred since they are inexpensive and widely available.

In the nitrogen-containing compound (8), R⁴ and R⁵ each independently represent hydrogen, C₁-C₁₂ alkyl, C₁-C₂₀ aralkyl, formyl, or C₂-C₁₂ acyl, in particular, hydrogen, methyl, ethyl, propyl, octyl, benzyl, naphthylmethyl, phenylethyl, formyl, acetyl, or benzoyl. R⁴ and R⁵ may bond with each other to form a ring. Examples of cyclic nitrogen-containing compound include pyrrolidine, piperidine, phthalimide, and derivatives thereof.

R⁴ and R⁵ are each preferably hydrogen or a group convertible to hydrogen, which is effective as the intermediate of serotonin agonist receptors. Thus, R⁴ and R⁵ are each hydrogen or phthalimide.

In the nitrogen-containing compound (8), Y¹ represents hydrogen or alkali metal. For example, Y¹ is hydrogen, lithium, sodium, or potassium.

The nitrogen-containing compound (8) is preferably inexpensive ammonia or potassium phthalimide.

The reaction of this step may be carried out with or without a reaction solvent. The reaction solvent used in this step is not particularly limited but is preferably a protic solvent or an aprotic solvent. Examples of the protic solvent include water and alcohol solvents such as methanol, ethanol, isopropanol, and n-butanol. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, and 1,1,1,-trichloroethane; and aprotic polar solvents such as dimethylformamide, N-methylpyrrolidone, and hexamethylphosphoric triamide. These solvents may be used alone or in combination.

The reaction temperature of this step is in the range of -80 to 150°C. Preferably, the reaction temperature is in the range of 20 to 120°C to increase the yield.

When ammonia is used as the nitrogen-containing compound, a pressure-resistant closed reactor, such as an autoclave, is preferably used to prevent evaporation of ammonia.

In this step, the completion of the reaction is detected by confirming the absence of the optically active sulfonyloxymethylchroman derivative (7) by analysis such as thin-layer chromatography, highperformance liquid chromatography, or gas chromatography.

In order to isolate the optically active aminomethylchroman derivative (9) prepared by the reaction of this step, a typical work up may be carried out. For example, a diluted acid, such as diluted hydrochloric acid, may be added after the completion of the reaction, followed by extraction using a common extraction solvent such as ethyl acetate, diethylether, methylene chloride, toluene, or hexane.

The obtained extract is, for example, heated under a reduced pressure to remove the reaction solvent and the extraction solvent to obtain the target substance. The target substance obtained is nearly pure but may be purified by crystallization, fractional distillation, or column chromatography, to increase the purity.

In the formulae representing the optically active sulfonyloxymethylchroman derivative (7) and the optically active aminomethylchroman derivative (9), the asterisked carbon atom is asymmetric.

The reaction of this step progresses with retention of the configuration at the asymmetric carbon. In particular, (R) isomers of the optically active aminomethylchroman derivative (9) are obtained from (R) isomers of the optically active sulfonyloxymethylchroman derivative (7). In general, in producing the optically active aminomethylchroman derivative (9) as the intermediates for serotonin receptor agonists, the (R) isomers of the optically active aminomethylchroman derivative (9) are preferably formed from (R) isomers of the optically active sulfonyloxymethylchroman derivative (7). In this manner, pharmaceutical intermediates disclosed in European Patent No. 707007 can be derived, for example.

### Step H

Optically active 2-halomethylchroman (11): 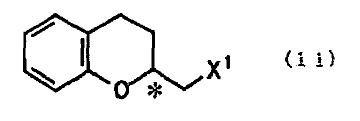 is reacted with a nitrogen-containing compound (8): 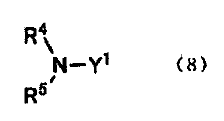 to produce an optically active aminomethylchroman derivative (9):

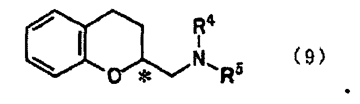

The optically active 2-halomethylchroman (11) used in this step is, for example, the optically active 2-halomethylchroman (11) prepared in Step F. Thus, X¹ is halogen, and preferably chlorine.

In the nitrogen-containing compound (8) used in this step, R⁴ and R⁵ each independently represent hydrogen, C₁-C₁₂ alkyl, C₁-C₂₀ aralkyl, formyl, or C₂-C₁₂ acyl. In particular, R⁴ and R⁵ each independently represent hydrogen, C₁-C₁₂ alkyl, C₁-C₂₀ aralkyl, formyl, or C₂-C₁₂ acyl, in particular, hydrogen, methyl, ethyl, propyl, octyl, benzyl, naphthylmethyl, phenylethyl, formyl, acetyl, or benzoyl. R⁴ and R⁵ may bond with each other to form a ring. Preferable examples of cyclic nitrogen-containing compound include pyrrolidine, piperidine, phthalimide, and derivatives thereof.

R⁴ and R⁵ are each preferably hydrogen or a group convertible to hydrogen. Thus, preferably, R⁴ and R⁵ are each hydrogen or phthalimide.

In the nitrogen-containing compound (8), Y¹ represents hydrogen or alkali metal. For example, Y¹ is hydrogen, lithium, sodium, or potassium.

The nitrogen-containing compound (8) is preferably inexpensive ammonia or potassium phthalimide.

Although the reaction of this step may be carried out without any reaction solvent, a reaction solvent may be used to dissolve materials and increase the reaction yield. The reaction solvent used in this step is not particularly limited but is preferably a protic solvent or an aprotic solvent. Examples of the protic solvent include water and alcohol solvents such as methanol, ethanol, isopropanol, and n-butanol. Examples of the aprotic solvent include hydrocarbon solvents such as benzene, toluene, n-hexane, and cyclohexane; ether solvents such as diethylether, tetrahydrofuran (THF), 1,4-dioxane, methyl t-butylether, dimethoxyethane, and ethylene glycol dimethylether; halogenated solvents such as methylene chloride, chloroform, and 1,1,1,-trichloroethane; and aprotic polar solvents such as dimethylformamide, N-methylpyrrolidone, and hexamethylphosphoric triamide. These solvents may be used alone or in combination.

The reaction temperature of this step is in the range of -80 to 150°C. Preferably, the reaction temperature is in the range of 20 to 120°C to increase the yield.

When ammonia is used as the nitrogen-containing compound, a pressure-resistant closed reactor, such as an autoclave, is preferably used to inhibit evaporation of ammonia.

In this step, the completion of the reaction is detected by confirming the absence of the optically active 2-halomethylchroman (11) by analysis such as thin-layer chromatography, highperformance liquid chromatography, or gas chromatography.

In order to isolate the optically active aminomethylchroman derivative (9) prepared by the reaction of this step, a typical work up may be carried out. For example, a diluted acid, such as diluted hydrochloric acid, may be added after the completion of the reaction, followed by extraction using a common extraction solvent such as ethyl acetate, diethylether, methylene chloride, toluene, or hexane.

The obtained extract is, for example, heated under a reduced pressure to remove the reaction solvent and the extraction solvent to obtain the target substance. The target substance obtained is nearly pure but may be purified by crystallization, fractional distillation, or column chromatography, to increase the purity.

In the formulae representing optically active 2-halomethylchroman (11) and the optically active aminomethylchroman derivative (9), the asterisked carbon atom is asymmetric.

The reaction of this step progresses with retention of the configuration at the asymmetric carbon. In particular, (R) isomers of the optically active aminomethylchroman derivative (9) are obtained from (R) isomers of optically active 2-halomethylchroman (11). In general, in producing the optically active aminomethylchroman derivative (9) as the intermediates for serotonin receptor agonists, the (R) isomers of the optically active aminomethylchroman derivative (9) are preferably formed from (R) isomers of optically active 2-halomethylchroman (11). In this manner, pharmaceutical intermediates disclosed in European Patent No. 707007 can be derived, for example.

The cyclic hemiacetal derivative (1): 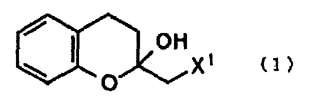 that can be efficiently produced by Step A of the present invention is a novel compound not disclosed in any literatures. Based on the investigations of the present inventors, the cyclic hemiacetal derivative (1), which is useful as a pharmaceutical intermediate, has been invented by the above-described method. In the cyclic hemiacetal derivative (1), X¹ represents halogen, and preferably chlorine.

The optically active halohydrin derivative (3): 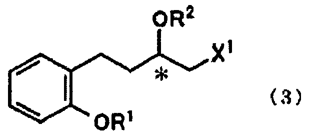 is a novel compound not disclosed in any literatures. The optically active halohydrin derivative (3) can be easily derived from the optically active halohydrin (5), which is efficiently synthesized in Step B of the present invention, by acylating or silylating the hydroxyl groups using a known method (e.g., a method disclosed in Greene, Protective Groups in Organic Synthesis, 2nd ed., John Wiley & Sons, inc.) Based on the investigations of the present inventors, the optically active halohydrin derivative (3), which is useful as a pharmaceutical intermediate, has been invented by the above-described method.

In the optically active halohydrin derivative (3), X¹ represents halogen, and preferably chlorine.

R¹ and R² each independently represent hydrogen, C₁-C₁₂ alkylsulfonyl, C₆-C₁₂ arylsulfonyl, C₁-C₁₀ acyl, or C₃-C₁₂ silyl. Preferably, R¹ and R² each independently represent hydrogen, alkylsulfonyl, or arylsulfonyl, and more preferably hydrogen or alkylsufonyl.

Specific examples thereof include hydrogen, methanesulfonyl, benzenesulfonyl, p-toluenesulfonyl, nitrobenzenesulfonyl, methoxybenzenesulfonyl, trifluoromethanesulfonyl, formyl, acetyl, propyonyl, benzoyl, trimethylsilyl, and tert-butyldimethylsilyl. Hydrogen and methanesulfonyl are particularly preferred.

The asterisked carbon atom is asymmetric. The configuration of the asymmetric carbon is preferably (S) when optically active halohydrin derivative (3) is used as the intermediates for serotonin receptor agonists. In this manner, pharmaceutical intermediates disclosed in European Patent No. 707007 can be derived, for example.

### (R)-2-Halomethylchroman (12):

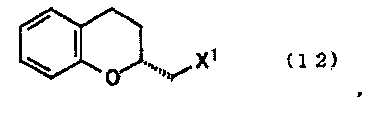

which can be efficiently produced by Step F of the present invention, is a novel compound not disclosed in any literatures. Based on the investigations of the present inventors, (R)-2-halomethylchroman (12), which is useful as a pharmaceutical intermediate, has been invented by the above-described method. In the (R)-2-halomethylchroman (12), X¹ represents halogen, and preferably chlorine.

The present invention will now be described in detail by way of examples. The present invention is not limited by these examples.

### EXAMPLE 1 2-Chloromethyl-2-hydroxychroman

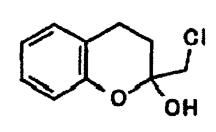

Under a nitrogen gas atmosphere, dihydrocoumarin (15g, 101.2 mmol, manufactured by Wako Pure Chemical Industries, Ltd.), sodium chloroacetate (17.6 g, 151.8 mmol, manufactured by Nacalai Tesque Inc.), and triethylamine (10.2 g, 101.2 mmol) were dissolved in tetrahydrofuran (300 mL) and the resulting solution was cooled to an outside temperature of 0°C. t-Butylmagnesium chloride (1.6 M THF-toluene (2.5:1) solution, 190 mL, 303.7 mmol) was added to the resulting solution over about 1.5 hours at an inside temperature of 2 to 6°C. Subsequently, the mixture was stirred for two hours while maintaining the outside temperature at 0°C. The resulting reaction mixture was added to 2.5 M hydrochloric acid (400 mL), and stirred for two hours at room temperature. The resulting reaction mixture was extracted with ethyl acetate (100 mL × three times). The extract was washed with saturated sodium bicarbonate aqueous solution (80 mL, once) and then with a saturated sodium chloride aqueous solution (80 mL, once), dried over anhydrous sodium sulfate, and filtered. The filtrate was condensed to obtain an yellow oil (18.2 g). The crude product was purified by column chromatography (silica gel, ethyl acetate:hexane = 1:7) to obtain an yellow oil of 2-chloromethyl-2-hydroxychroman (16.2 g, 80%). ¹H-NMR (400 MHz) δ 2.00-2.08 (2H, m), 2.22-2.28 (2H, m), 2.85-2.91 (1H, m), 3.14-3.26 (1H, m), 3.35 (1H, d, J = 1.96 Hz), 3.87 (2H, dd, J = 11.5, 33.5 Hz), 6.97-7.12 (2H, m), 7.2-7.3 (2H, m).
¹³C-NMR (100 MHz) δ 21.1, 28.2, 51.3, 94.9, 117.0, 121.2, 121.5, 127.5, 129.1, 161.8

### EXAMPLE 2 (2S)-1-Chloro-4-(2-hydroxyphenyl)butan-2-ol

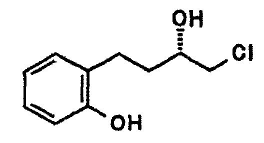

5 ml of a liquid medium (pH: 7.0) containing 40 g of glucose, 3 g of yeast extract, 6.5 g of diammonium hydrogen phosphate, 1 g of dipotassium hydrogen-phosphate, 0.8 g of magnesium sulfate heptahydrate, 60 mg of zinc sulfate heptahydrate, 90 mg of iron sulfate heptahydrate, 5 mg of copper sulfate pentahydrate, 10 mg of manganese sulfate tetrahydrate, and 100 mg of sodium chloride per liter was distributed in 5 ml portions into a large size test tube and was steam-sterilized at 120°C for 20 minutes. The liquid broth portion was aseptically inoculated with a loopful of Candida magnoliae IF0705, and was incubated with shaking at 30°C for 24 hours. Upon completion of the incubation, the cells were collected from the culture by centrifugation and were suspended in 0.5 ml of a 100 mM phosphate buffer solution (pH: 6.5) containing 40 mg of glucose. To the suspension, 5 mg of 1-chloromethyl-1-hydroxychroman was added, and the reaction was continued at 30°C for 24 hours. Subsequently, the reaction mixture was thoroughly mixed with 5 ml of ethyl acetate, and a part of the organic phase was analyzed under HPLC conditions described below. As a result, 1.5 mg of (2S)-1-chloro-4-(2-hydroxyphenyl)butan-2-ol (optical purity: 98.5% ee) was obtained.

### EXAMPLE 3 (2S)-1-Chloro-4-(2-hydroxyphenyl)butan-2-ol

The same reaction as in EXAMPLE 2 was carried out using a culture broth of Candida maris IFO10003 prepared as in EXAMPLE 2 with the same culture medium. As a result, 0.8 mg of (2S)-1-chloro-4-(2-hydroxyphenyl)butan-2-ol (optical purity: 99.5% ee) was obtained.

### EXAMPLE 4 (2S)-1-Chloro-4-(2-hydroxyphenyl)butan-2-ol

A 50 ml of a liquid broth (pH 7.0) containing 16 g of bacto-tryptone, 10 g of yeast extract, and 5 g of sodium chloride per liter was placed in a 500 ml Sakaguchi flask and steam-sterilized at 120°C for 20 minutes. The liquid broth was asceptically inoculated with a loopful of Escherichia coli HB101 (pNTS1G), accession number FERM BP05835 (deposited with International Patent Organism Depositary, National Institute of Advanced Industrial Science and Technology, Tsukuba Central 6, 1-1-1 Higashi, Tsukuba, Ibaraki, Japan, since February 24, 1997). After 24 hours of culture with shaking at 37°C, the culture broth was centrifuged to collect a cellular fraction, and the cells were suspended in 50 ml of 100 mM phosphate buffer solution (pH 7.0). To the suspension, 500 mg of 1-chloromethyl-1-hydroxychroman, 550 mg of glucose, and 10 mg of NADP⁺ were added, and the mixture was allowed to react at 30°C for 24 hours with stirring while maintaining pH of the reaction mixture at 6.5 by adding a 5M sodium hydroxide aqueous solution. Subsequently, the reaction mixture was extracted with ethyl acetate (100 ml × twice). The organic phase was dried over anhydrous sodium sulfate and removed by filtration. The solvent was distilled off under a reduced pressure. The residue was purified by silica gel column chromatography to obtain 480 mg of (2S)-1-chloro-4-(2-hydroxyphenyl)butan-2-ol (yield: 95%). The optical purity determined by the method described in EXAMPLE 1 was 98.5% ee.
¹H-NMR (400 MHz) δ 1.74-1.92 (2H, m), 2.71-2.90 (2H, m), 3.16 (1H, br), 3.45-3.62 (2H, m), 3.77 (1H, br), 6.79-6.88 (3H, m), 7.09-7.25 (2H, m).
¹³C-NMR (100 MHz) **δ** 25.4, 34.4, 49.8, 70.2, 116.1, 120.9, 126.9, 127.7, 130.5, 154.0.

### EXAMPLE 5 (2S)-1-Chloro-4-(2-hydroxyphenyl)butan-2-ol

500 mg of 1-chloromethyl-1-hydroxychroman, 550 mg of glucose, and 20 mg of NADP were added to 50 ml of a suspension of Escherichia coli HB101 (pNTFPG), accession number FERM BP-7117 (deposited with International Patent Organism Depositary, National Institute of Advanced Industrial Science and Technology, Tsukuba Central 6, 1-1-1 Higashi, Tsukuba, Ibaraki, Japan, since April 11, 2000) prepared as in EXAMPLE 2 using the same culture medium. The mixture was allowed to react at 30°C for 48 hours with stirring while maintaining pH of the reaction mixture at 6.5 using a 5M sodium hydroxide aqueous solution. Subsequently, 480 mg of (2S)-1-chloro-4-(2-hydroxyphenyl)butan-2-ol (yield: 95%) was obtained by the same process as in EXAMPLE 3. The optical purity determined by the process described in EXAMPLE 1 was 99.5% ee.

### EXAMPLE 6 (R)-2-Hydroxymethylchroman

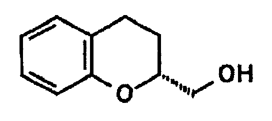

To 30 mL of a methanol solution of (2S)-1-chloro-4-(2-hydroxyphenyl)butan-2-ol (3.5 g, 17.4 mmol), a 2M sodium hydroxide aqueous solution (43 mL, 87.2 mmol) was gradually added dropwise at room temperature, followed by one hour of stirring. The reaction mixture was extracted with ethyl acetate (50 mL × three times), dried over anhydrous sodium sulfate, and filtered. The filtrate was condensed to obtain a white solid (2.90 g). The crude product was purified by column chromatography (silica gel, ethyl acetate:hexane = 1:7) to obtain an oily pale-yellow mixture of (R)-2-hydroxymethylchroman and 6,7,8,9-tetrahydro-5H-benzo[a]-cyclohepten-7-ol (2.74 g, 96%). The crude product was analyzed by highperformance liquid chromatography. The content of 2-hydroxymethylchroman was 80% (yield: 77%). The next reaction was directly performed without isolation or refining.
¹H-NMR (400 MHz) δ 1.80-1.97 (2H, m), 2.2 (1H, br), 2.75-2.93 (2H, m), 3.73-3.99 (2H, m), 4.10-4.14 (1H, m), 3.77 (1H, br), 6.81-6.87 (2H, m), 7.00-7.15 (2H, m).

### EXAMPLE 7 (R)-2-Methanesulfonyloxymethylchroman

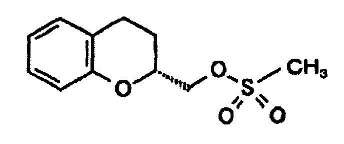

Under a nitrogen atmosphere, (R)-2-hydroxymethylchroman (2.74 g, 16.7 mmol, content: 80%) prepared in EXAMPLE 6 and triethylamine (2.5 g, 25.0 mmol) were dissolved in tetrahydrofuran. The solution was cooled to an outside temperature of 0°C. Methanesulfonyl chloride (2.3 g, 20.0 mmol) was gradually added dropwise to the solution, followed by one hour of stirring. To the reaction mixture, a saturated ammonium chloride aqueous solution (30 mL) was added, and the mixture was extracted with ethyl acetate (50 mL x three times). The extract was washed once with a saturated sodium chloride aqueous solution (50 mL), dried over anhydrous sodium sulfate, and filtered. The filtrate was condensed to obtain (R)-2-methanesulfonyloxymethylchroman as a white solid (4.00 g, 95%).
¹H-NMR (400 MHz) δ 1.83-1.96 (1H, m), 2.01-2.10 (1H, m), 2.77-2.94 (2H, m), 3.10 (1H, s), 4.27-4.29 (1H, m), 4.36-4.46 (2H, m), 6.79-6.89 (2H, m), 7.02-7.16 (2H, m).

### EXAMPLE 8 (R)-2-p-Toluenesulfonyloxymethylchroman

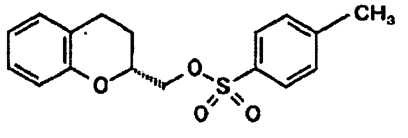

(R)-2-p-toluenesulfonyloxymethylchroman as a white solid (60%) was obtained as in EXAMPLE 7 but with p-toluenesulfony chloride instead of methanesulfony chloride. ¹H-NMR (400 MHz) δ 1.76-1.82 (1H, m), 1.97-2.03 (1H, m), 2.45 (3H, s), 2.71-2.82 (2H, m), 4.16-4.23 (3H, m), 6.68 (1H, d), 6.83 (1H, dt, J = 1.5, 7.5 Hz), 7.00-7.07 (2H, m), 7.35 (2H, d, J = 7.5 Hz), 7.82 (2H, d, J = 8.3 Hz).

### EXAMPLE 9 (R)-2-Aminomethylchroman

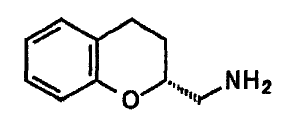

In an autoclave, (R)-2-methanesufonyloxymethylchroman (10.0 g, 41.3 mmol, content: 80%) was dissolved in tetrahydrofuran (15 mL), and the solution was cooled to an outside temperature of -78°C. Liquid ammonia (120 mL, 5.4 mol) was introduced into the autoclave. The autoclave was sealed and heated to an outside temperature of 65°C (inside temperature: 56.4 to 57.6°C, inside pressure 2.0 MPa), followed by 24 hours of stirring. After the temperature and the pressure inside the autoclave were returned to normal, a 1M HCl aqueous solution (40 mL) was added, followed by extraction with ethyl acetate (70 mL × three times). To the remaining water layer, 50 mL of 30 wt% aqueous sodium hydroxide was added, and the mixture was extracted with ethyl acetate (70 mL × three times). The extract was washed once with saturated aqueous sodium chloride (50 mL), dried over anhydrous sodium sulfate, and filtered. The extract was condensed to obtain (R)-2-aminomethylchroman as transparent pale-yellow liquid (4.24 g, 87%).
¹H-NMR (400 MHz) δ 1.63 (1H, br), 1.70-1.81 (1H, m), 1.90-1.97 (1H, m), 2.71-2.92 (4H, m), 3.92-3.98 (1H, m), 6.78-6.86 (2H, m), 6.97-7.14 (2H, m).

### EXAMPLE 10 (S)-2-[4-Chloro-3-(methanesulfonyloxy)butyl]phenylmethanesufonate

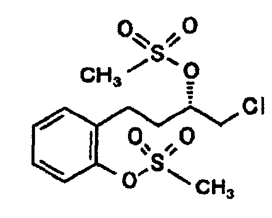

Under a nitrogen gas atmosphere, (2S)-1-chloro-4-(2-hydroxyphenyl)butan-2-ol (1.41 g, 8.6 mmol) and triethylamine (3.90 g, 38.6 mmol) were dissolved in tetrahydrofuran (30 mL), and the solution was cooled to an outside temperature of 0°C. Methanesulfonyl chloride (3.24, 28.3 mmol) was gradually added to the solution, followed by one hour of stirring while maintaining the outside temperature at 0°C. Saturated aqueous ammonium chloride was added to the reaction mixture, and the resulting reaction mixture was extracted with ethyl acetate (30 mL × three times). The extract was washed once with saturated aqueous sodium chloride (30 mL), dried over anhydrous sodium sulfate, and filtered. The filtrate was condensed to obtain (S)-2-[4-chloro-3-(methanesulfonyloxy)butyl]phenylmethanesufonate (2.57 g, 84%) as a solid.
¹H-NMR (400 MHz) δ 2.13 (2H, dt, J = 5.8, 8.3 Hz), 2.76-2.85 (1H, m), 2.87-2.94 (1H, m), 3.13 (3H, s), 3.26 (1H, s), 3.70-3.79 (2H, m), 4.86-491 (2H, tt, J = 5.8, 5.8 Hz), 7.25-7.33 (4H, m).

### EXAMPLE 11 (R)-2-Chloromethylchroman

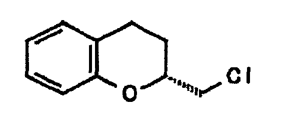

In a mixed solvent of ethanol (150 mL) and water (50 mL), (S)-2-[4-chloro-3-(methanesulfonyloxy)butyl]phenylmethanesufonate (10.8 g, 30.4 mmol) was dissolved. To the solution, a 3M potassium hydroxide aqueous solution (100 mL) was gradually added dropwise at room temperature, followed by 40 hours of stirring. Saturated aqueous ammonium chloride (100 mL) was added to the reaction mixture, and ethanol was removed with a rotary evaporator. The residue was extracted with ethyl acetate (100 mL × three times). The extract was washed once with saturated aqueous sodium chloride (100 mL), dried over anhydrous sodium sulfate, and filtered. The filtrate was condensed to obtain a solid (3.6 g). The crude product was purified by column chromatography (silica gel, ethyl acetate:hexane = 1:10) to obtain liquid (R)-2-chloromethylchroman (2.50 g, 45%).
¹H-NMR (400 MHz) δ 1.85-1.95 (1H, m), 2.12-2.18 (1H, m), 2.76-2.93 (2H, m), 3.64-3.77 (2H, m), 4.20-4.26 (2H, m), 6.83-6.88 (2H, m), 7.04-7.12 (2H, m).

### EXAMPLE 12 (R)-2-N-Phthaloylaminomethylchroman

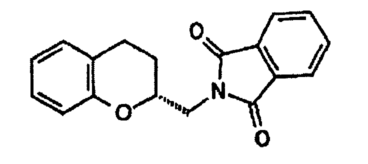

Under a nitrogen atmosphere, (R)-2-chloromethylchroman (150 mg, 0.82 mmol) and potassium phthalimide (304 mg, 1.64 mmol) were dissolved in dimethylformamide (8 mL), followed by 20 hours of stirring at 90°C. To the reaction mixture, saturated aqueous ammonium chloride (10 mL) was added, and the mixture was extracted with ethyl acetate (20 mL × three times). The extract was washed once with saturated aqueous sodium chloride (20 mL), dried over anhydrous sodium sulfate, and filtered. The filtrate was condensed to give a solid (220 mg). The crude product was purified by silica gel chromatography (silica gel, ethyl acetate:hexane = 1:5) to obtain (R)-2-N-phthaloylaminomethylchroman as a white solid (141 mg, 57%).
¹H-NMR (400 MHz) δ 1.74-1.92 (2H, m), 2.71-2.90 (2H, m), 3.16 (1H, br), 3.45-3.62 (2H, m), 3.77 (1H, br), 6.79-6.88 (3H, m), 7.09-7.25 (2H, m), 7.71-7.76 (2H, m), 7.86-7.90 (2H, m).

### EXAMPLE 13 (R)-2-N-Phthaloylaminomethylchroman

(R)-2-N-phthaloylaminomethylchroman was prepared as in EXAMPLE 12 but with (R)-2-hydroxymethylchroman (150 mg, 0.62 mmol) (yield: 60%).

### Industrial Applicability

The present invention provides a practical process of synthesizing optically active chroman derivatives from dihydrocoumarin, the process including three to five steps. Important intermediates for synthesizing the optically active chroman derivatives are also provided.

## Claims

1. A cyclic hemiacetal derivative represented by general formula (1): 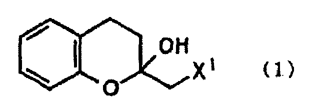 (wherein X¹ represents halogen).

2. A method for producing a hemiacetal derivative represented by general formula (1): 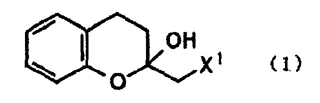 (wherein X¹ represents halogen), the method comprising reacting dihydrocoumarin with an enolate prepared from a base and a haloacetic acid derivative represented by general formula (2): 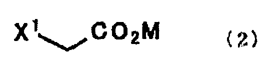 (wherein X¹ represents halogen, M represents hydrogen, alkali metal, or halogenated alkali earth metal), followed by acid-treatment.

3. The method according to claim 2, wherein the base is tert-butylmagnesium chloride.

4. The method according to claim 2 or 3, wherein dihydrocoumarin is reacted with the enolate in the presence of an amine.

5. An optically active halohydrin derivative represented by general formula (3): 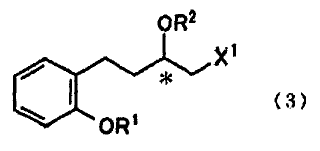 (wherein X¹ represents halogen; R¹ and R² each independently represent hydrogen, C₁-C₁₂ alkylsulfonyl, C₆-C₁₂ arylsulfonyl, C₁-C₁₀ acyl, or C₃-C₁₂ silyl; and the asterisked carbon atom is asymmetric).

6. The optically active halohydrin derivative according to claim 5, wherein R¹ and R² each independently represent hydrogen, C₁-C₁₂ alkylsulfonyl, or C₆-C₁₂ arylsulfonyl.

7. The optically active halohydrin derivative according to claim 5 or 6, wherein the asymmetric carbon has the (S) configuration.

8. A method for producing an optically active halohydrin derivative represented by general formula (5): 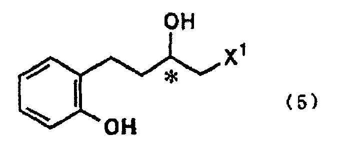 (wherein X¹ represents a halogen atom and the asterisked carbon is asymmetric), the method comprising stereoselectively reducing a selected one of a cyclic hemiacetal derivative represented by general formula (1) 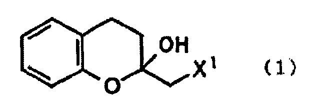 (wherein X¹ represents a halogen atom), a haloketone derivative represented by general formula (4) 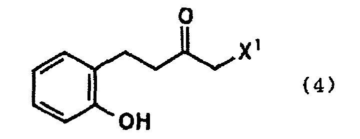 (wherein X¹ represents a halogen atom), and a mixture of these, by causing an enzyme capable of asymmetrically reducing the same to act thereon.

9. The production method according to claim 8, wherein the product optically active halohydrin has the S absolute configuration.

10. The production method according to claim 8 or 9, wherein the enzyme resides in a cellular fraction or a culture of a microorganism or in a processed matter thereof, the organism belonging to the genus Candida.

11. The production method according to claim 10, wherein the enzyme resides in a culture broth, a cellular fraction, or a processed matter of a microorganism belonging to the species Candida magnoliae or Candida maris.

12. The production method according to claim 8 or 9, wherein the enzyme resides in a cell transformed with a plasmid having a DNA coding a reducing enzyme derived from a microorganism belonging to the genus Candida and a DNA coding an enzyme that regenerates a coenzyme on which the enzyme depends.

13. The production method according to claim 12, wherein the transformant cell is Escherichia coli HB101 (pNTS1G), accession number FERM BP-5835, or Escherichia coli HB101 (pNTFPG), accession number FERM BP-7117.

14. A method for producing an optically active 2-hydroxymethylchroman represented by formula (6): 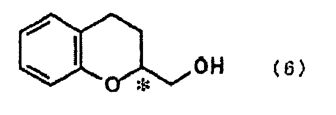 (wherein the asterisked carbon atom is asymmetric), which comprises cyclizing an optically active halohydrin represented by general formula (5): 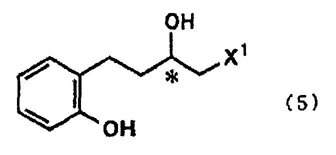 (wherein X¹ represents halogen and the asterisked carbon atom is asymmetric) with a base.

15. The method according to claim 14, wherein the base is a metal hydroxide.

16. The method according to claim 14 or 15, wherein water is used as a reaction solvent during the cyclizing.

17. The method according to claim 14, 15, or 16, wherein the asymmetric carbon of the optically active halohydrin has the (S) configuration, and the asymmetric carbon of the 2-hydroxymethylchroman has the (R) configuration.

18. A method for producing an optically active sulfonyloxymethylchroman derivative represented by general formula (7) 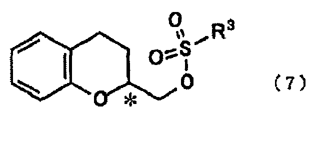 (wherein R³ represents and the asterisked carbon atom is asymmetric), which comprises cyclizing an optically active halohydrin represented by general formula (5): 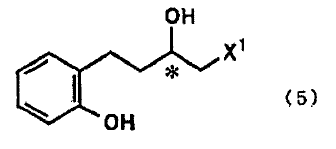 (wherein X¹ represents halogen and the asterisked carbon atom is asymmetric) with a base, and reacting the resulting product with a sulfonylating agent.

19. The method according to claim 18, wherein the sulfonylating agent is added directly after the completion of the reaction of the optically active halohydrin with the base.

20. The method according to claim 18 or 19, wherein the base is a metal hydroxide or a metal hydride.

21. The method according to claim 18, 19, or 20, wherein tetrahydrofuran is used as the cyclization reaction solvent.

22. The method according to claim 18, 19, 20, or 21, wherein water is used as the cyclization reaction solvent or is contained in a mixed solvent used as the cyclization reaction solvent.

23. The method according to claim 18, 19, 20, 21, or 22, wherein the sulfonylating agent is a methanesulfonyl chloride, a p-toluenesulfonyl chloride, or a benzenesulfonyl chloride.

24. The method according to claim 18, 19, 20, 21, 22, or 23, wherein the reaction with the sulfonylating agent is carried out in the presence of a tertiary amine.

25. The method according to claim 18, 19, 20, 21, 22, 23, or 24, wherein the asymmetric carbon of the optically active halohydrin has the (S) configuration and the asymmetric carbon of the optically active sulfonyloxymethylchroman derivative has the (R) configuration.

26. The method according to claim 18, 19, 20, 21, 22, 23, 24, or 25, wherein R³ represents methyl, phenyl, or p-tolyl.

27. A method for producing an optically active aminomethylchroman derivative represented by general formula (9): 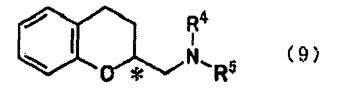 (wherein R⁴ and R⁵ each independently represent hydrogen, C₁-C₁₂ alkyl, C₁-C₂₀ aralkyl, formyl, or C₂-C₁₂ acyl, and the asterisked carbon atom is asymmetric), which comprises reacting an optically active sulfonyloxymethylchroman derivative represented by general formula (7): 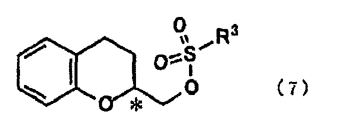 (wherein R³ represents C₁-C₁₂ alkyl or C₆-C₁₂ aryl, and the asterisked carbon atom is asymmetric) with a nitrogen-containing compound represented by general formula (8): 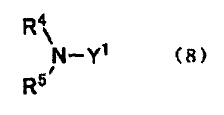 (wherein R⁴ and R⁵ each independently represent hydrogen, C₁-C₁₂ alkyl, C₁-C₂₀ aralkyl, formyl, or C₂-C₁₂ acyl, and Y¹ represents hydrogen or an alkali metal).

28. The method according to claim 27, wherein the optically active sulfonyloxymethylchroman derivative is prepared by cyclizing an optically active halohydrin represented by general formula (5): 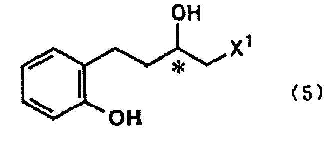 (wherein X¹ represents halogen and the asterisked carbon atom is asymmetric) with a base and then reacting the resulting product with a sulfonylating agent.

29. The method according to claim 27 or 28, wherein the nitrogen-containing compound is ammonia.

30. The method according to claim 27 or 28, wherein the nitrogen-containing compound is potassium phthalimide.

31. The method according to claim 27, 28, 29, or 30, wherein the asymmetric carbon of the optically active halohydrin has the (S) configuration and the asymmetric carbon of the optically active aminomethylchroman derivative has the (R) configuration.

32. A method for producing an optically active 2-halomethylchroman compound represented by general formula (11) 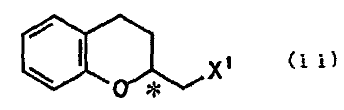 (wherein X¹ represents halogen and the asterisked carbon atom is asymmetric), which comprises reacting an optically active disulfonate derivative represented by general formula (10) 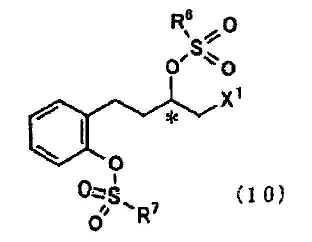 (wherein X¹ is halogen; R⁶ and R⁷ are each independently C₁-C₁₂ alkyl or C₆-C₁₂ aryl; and * is the same as above) with a base in a protic solvent or a mixed solvent containing a protic solvent so as to perform solvolysis of the sulfonyl group and cyclization.

33. The method according to claim 32, wherein the optically active disulfonate derivative is prepared by reacting an optically active halohydrin represented by general formula (5): 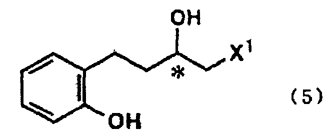 (wherein X¹ represents halogen and the asterisked carbon atom is asymmetric) with a sulfonylating agent.

34. The method according to claim 33, wherein the sulfonylating agent is a methanesulfonyl chloride, a p-toluenesulfonyl chloride, or a benzenesulfonyl chloride.

35. The method according to claim 33 or 34, wherein the reaction with the sulfonylating agent is carried out in the presence of a tertiary amine.

36. The method according to claim 32, 33, 34, or 35, wherein the asymmetric carbon of the optically active halohydrin has the (S) configuration.

37. The method according to claim 32, 33, 34, 35, or 36, wherein at least one of R⁶ and R⁷ is methyl, phenyl, or tolyl.

38. The method according to claim 32, 33, 34, 35, 36, or 37, wherein the protic solvent is water or methanol.

39. The method according to claim 32, 33, 34, 35, 36, 37, or 38, wherein the base is a metal hydroxide.

40. The method according to claim 32, 33, 34, 35, 36, 37, 38, or 39, wherein the base is an alkali metal alkoxide.

41. The method according to claim 32, 33, 34, 35, 36, 37, 38, 39, or 40, wherein the asymmetric carbon of the optically active disulfonate derivative has the (S) configuration and the asymmetric carbon of the optically active halomethylchroman has the (R) configuration.

42. A method for producing an optically active aminomethylchroman derivative represented by general formula (9): 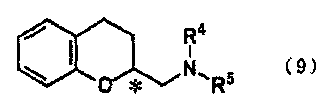 (wherein R⁴ and R⁵ each independently represent hydrogen, C₁-C₁₂ alkyl, C₁-C₂₀ aralkyl, formyl, or C₂-C₁₂ acyl, and the asterisked carbon atom is asymmetric), the method including reacting an optically active 2-halomethylchroman represented by general formula (11) 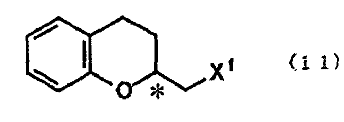 (wherein X¹ represents halogen and the asterisked carbon atom is asymmetric) with a nitrogen-containing compound represented by general formula (8): 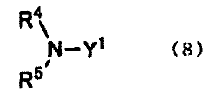 (wherein R⁴ and R⁵ each independently represent hydrogen, C₁-C₁₂ alkyl, C₁-C₂₀ aralkyl, formyl, or C₂-C₁₂ acyl, and Y¹ represents hydrogen or an alkali metal).

43. The method according to claim 42, wherein the optically active 2-halomethylchroman is prepared by reacting an optically active disulfonate derivative represented by general formula (10) 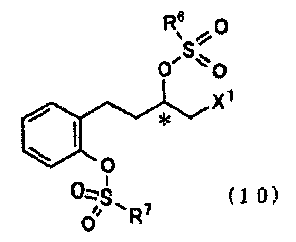 (wherein X¹ represents halogen; R⁶ and R⁷ are each independently C₁-C₁₂ alkyl or C₆-C₁₂ aryl; and the asterisked carbon atom is asymmetric) with a base in a protic solvent or a mixed solvent containing a protic solvent so as to perform solvolysis of sulfonyl group and cyclization.

44. The method according to claim 43, wherein the protic solvent is water or methanol.

45. The method according to claim 43 or 44, wherein the base is a metal hydroxide.

46. The method according to claim 43, 44, or 45, wherein the base is an alkali metal alkoxide.

47. The method according to claim 42, 43, 44, 45, or 46, wherein the nitrogen-containing compound is ammonia.

48. The method according to claim 42, 43, 44, 45, or 46, wherein the nitrogen-containing compound is potassium phthalimide.

49. The method according to claim 42, 43, 44, 45, 46, 47, or 48, wherein the asymmetric carbon of the optically active disulfonate derivative has the (S) configuration and the asymmetric carbon of the optically active aminomethylchroman derivative has the (R) configuration.

50. A (R)-2-halomethylchroman compound represented by general formula (12): 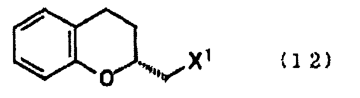 (wherein X¹ represents halogen).

51. The (R)-2-halomethylchroman compound according to claim 50, wherein X¹ is chlorine.
